# FASCICULE DE BREVET EUROPEEN

(11) **EP 0 858 800 B1**
(45) Date de publication et mention de la délivrance du brevet: **09.08.2000**
(21) Numéro de dépôt: 98400111.5
(22) Date de dépôt: 21.01.1998
(51) Int. Cl.: A61K 7/48

(54) **Emulsions eau-dans-huile à base de deux émulsionnants particuliers et applications cosmétiques**
Auf zwei spezifisische Emulgatoren basierende Wasser-in-Öl Emulsion, sowie kosmetische Verwendung dieser
Water in oil emulsions based on two specific emulsifiers and cosmetic uses thereof

(30) Priorité: 06.02.1997 FR 9701367
(43) Date de publication de la demande: 19.08.1998
(73) Titulaire: L'OREAL, 75008 Paris (FR)
(72) Inventeur: Hansenne, Isabelle, 75017 Paris (FR); De Chabannes, Karine, 45000 Orleans (FR)
(74) Mandataire: Miszputen, Laurent

(56) Documents cités:
- EP-A- 0 590 601
- EP-A- 0 633 018
- DE-A- 4 420 516
- GB-A- 2 242 358

## Description

La présente invention se rapporte à de nouvelles compositions du type eau-dans-huile, en particulier à de nouvelles compositions cosmétiques destinées à la protection de la peau et/ou des cheveux contre les rayonnements UV, et comprenant l'association de deux émulsionnants particuliers.

L'invention se rapporte également aux applications de ces compositions dans le domaine cosmétique susmentionné.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination d'UV-B, provoquent des érythèmes et des brûlures cutanées qui peuvent nuire au développement du bronzage naturel. Pour ces raisons ainsi que pour des raisons esthétiques, il existe une demande constante de moyens de contrôle de ce bronzage naturel en vue de contrôler ainsi la couleur de la peau ; il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.

De nombreuses compositions cosmétiques et/ou dermatologiques destinées à la photoprotection (UV-A et/ou UV-B) de la peau ont été proposées à ce jour.

Ces compositions antisolaires se présentent assez souvent sous la forme d'une émulsion de type huile-dans-eau (c'est à dire un support cosmétiquement et/ou dermatologiquement acceptable constitué d'une phase continue dispersante aqueuse et d'une phase discontinue dispersée huileuse) en raison notamment de leur toucher agréable (voisin de l'eau) et de leur présentation sous forme de lait ou de crème non gras.

Toutefois, un des inconvénients des émulsions huile-dans-eau est qu'elles perdent très facilement leur efficacité en protection UV dès lors qu'elles viennent en contact avec l'eau ; en effet, les filtres qu'elles contiennent dans leur phase aqueuse sont entraînés par l'eau, lors de baignades en mer ou en piscine par exemple, ou encore sous la douche ou lors de la pratique de sports nautiques et le pouvoir photoprotecteur global de ces compositions est fortement diminué.

C'est pourquoi, dans certains cas où une protection particulièrement élevée et durable est recherchée, comme pour les peaux des enfants ou encore pour les peaux sensibles, on préfère mettre en oeuvre des compositions eau-dans-huile qui présentent une bonne rémanence à l'eau et qui conservent ainsi un très bon pouvoir photoprotecteur même après plusieurs bains.

Pour de telles peaux, on cherche toujours en outre à minimiser les risques de sensibilisation en évitant au maximum la présence, au sein des formulations, d'agents susceptibles de provoquer des réactions de la peau : ainsi, on cherche à formuler des compositions qui puissent à la fois être exemptes d'agents conservateurs tout en présentant une stabilité optimale durant tout le temps de la conservation et de l'utilisation des produits.

Enfin, ces compositions étant notamment destinées à être appliquées sur la peau, elles doivent également présenter de bonnes propriétés cosmétiques, à savoir une bonne tenue, une bonne résistance à l'environnement immédiat, et dans le cas particulier des compositions solaires pour enfants une non-adhérence au sable avec lequel ils sont souvent en contact (plage, aires de jeux) : ces compositions doivent pouvoir également permettre un étalement facile et repérable.

On connaît dans le document EP-A-0633 018 des mélanges auto-émusionnables constitués d'un copolyol polydiméthylsiloxane polyoxypropyléné et polyoxyéthyléné et d'une huile de silicone permettant de préparer des émulsions huile dans eau stables par simple addition d'eau audit mélange. On connaît également dans le document EP-0590 601 des formulations pour le nettoyage de la peau sous forme d'émulsions huile dans eau contenant un polyéthylène glycol stéarate et/ou un polyéthylène glycol dioléate à 10-18 moles d'oxyde d'éthylène en présence d'un polysiloxane polyoxyalkyléné.

On connaît dans la demande de brevet GB 2 242 358 des émulsions de type eau-dans-huile stabilisées par des émulsionnants siliconés tels que les diméthicone copolyols qui sont bien connus pour leurs bonnes propriétés émulsionnantes eau-dans-huile.

On connaît enfin dans le document DE 442 0516 des émulsions de type eau-dans-huile stabilisées par des émulsionnants du type polyolpolyhydroxystéarate ayant un degré de polycondensation de l'acide polyhydroxystéarique avec le polyol étant de 2 à 20 et comprenant un mélange de polyglycerine constitué de monoglycérine , diglycérine, triglycérine, tétraglycérine, de pentaglycérine et d'oligoglycérine.

La Demanderesse a découvert de manière surprenante que l'utilisation, au sein de compositions comprenant au moins un polyalkyl polyéther siloxane convenablement sélectionné au sein des émulsionnants siliconés, d'un polymère particulier présentant des propriétés émulsionnantes eau-dans-huile, permettait d'obtenir des émulsions eau-dans-huile présentant une stabilité améliorée.

La présente invention a donc pour objet une nouvelle émulsion de type eau-dans-huile, stabilisée, caractérisée par le fait qu'elle comprend au moins un émulsionnant siliconé constitué d'un polyalkyl polyéther siloxane portant des chaînes polyoxyéthylènes et polyoxypropylènes greffées sur la chaîne principale et au moins un polymère du type ester d'acide gras de glycol polyoxyalkyléné ayant des propriétés émulsionnantes eau-dans-huile.

Les émulsions selon l'invention présentent les avantages d'être rémanentes à l'eau et d'être particulièrement stables dans le temps, même sans conservateur. De plus, les émulsions conformes à l'invention adhèrent très peu au sable et présentent de très bonnes propriétés cosmétiques.

La présente invention a également pour objet l'utilisation des émulsions définies ci-dessus comme, ou pour la fabrication de, compositions cosmétiques et/ou dermatologiques destinées à la protection de la peau et/ou des cheveux contre le rayonnement ultraviolet.

Un autre objet encore de la présente invention réside dans un procédé de traitement cosmétique pour la protection de la peau et/ou des cheveux contre le rayonnement ultraviolet, qui consiste essentiellement à appliquer sur ces derniers une quantité efficace d'une émulsion ou d'une composition cosmétique et/ou dermatologique telles que définies ci-dessus.

D'autres caractéristiques, aspects et avantages de la présente invention apparaîtront à la lecture de la description détaillée qui va suivre.

Selon une caractéristique essentielle de la présente invention, les compositions conformes à l'invention comprennent un émulsionnant siliconé spécifique constitué d'un polyalkyl polyéther siloxane portant des chaînes polyoxyéthylènes et polyoxypropylènes greffées sur la chaîne principale.

De préférence, cet émulsionnant siliconé est choisi parmi les composés de formule générale (I) suivante : 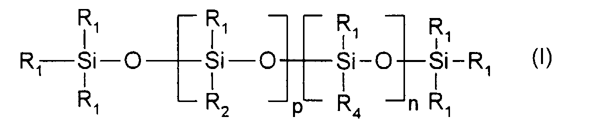 dans laquelle :
- R₁ et R₄, identiques ou différents, représentent un atome d'hydrogène, un radical alkyle linéaire ou ramifié en C₁-C₃₀ ou un radical phényle,
- R₂, identiques ou différents, représentent -(CₓH₂ₓ)-(OC₂H₄)ₐ-(OC₃H₆)_{b}-OR₃,
- R₃, identiques ou différents, sont choisis parmi un atome d'hydrogène, un radical alkyle, linéaire ou ramifié, ayant de 1 à 12 atomes de carbone, un radical acyle, linéaire ou ramifié ayant de 2 à 12 atomes de carbone,
- n varie de 1 à 1000,
- p varie de 1 à 30,
- a varie de 1 à 50,
- b varie de 1 à 50,
- x varie de 1 à 5.

Le poids moléculaire moyen en nombre de cet émulsionnant siliconé est en général supérieur ou égal à 15000 et de préférence compris entre 20 000 et 40 000.

Une première famille de polyalkyl polyéther siloxanes convenant particulièrement bien aux compositions selon l'invention est celle des composés répondant à la formule (I) ci-dessus pour lesquels les radicaux R₁ et R₄ sont identiques et représentent tous des radicaux méthyle et le radical R₃ représente l'hydrogène.

A titre d'exemple d'émulsionnant siliconé appartenant à cette famille, on peut citer le poly diméthyl / méthyl siloxane oxyéthyléné oxypropyléné (OE/OP 18/18) pour lequel n est 396 et p est 4, de poids moléculaire en nombre supérieur à 30 000 (nom CTFA : Cyclométhicone 90% Dimethicone copolyol 10%) vendu sous la dénomination commerciale de « Silicone Q₂3225C » par la société Dow Corning. Dans la définition ci-dessus et dans la suite du texte, OE représente une mole d'oxyde d'éthylène et OP représente une mole d'oxyde de propylène.

Une deuxième famille de polyalkyl polyéther siloxanes convenant particulièrement bien aux compositions selon l'invention est celle des composés répondant à la formule (I) ci-dessus pour lesquels les radicaux R₁ représentent tous des radicaux méthyle et les radicaux R₄ représentent tous des radicaux lauryle. Un émulsionnant siliconé particulièrement préféré de cette deuxième famille est le poly méthyllauryl / méthyl siloxane oxyéthyléné oxypropyléné (OE/OP 18/18) pour lequel n est 35 et p est 3, de poids moléculaire en nombre supérieur à 25 000 (nom CTFA : Laurylméthicone copolyol 91%, Isostéaryl alcohol 9%) vendu sous la dénomination commerciale « DC Q2-5200 » par la société Dow Corning. Le ou les émulsionnants siliconés sont généralement présents dans les compositions selon l'invention en une proportion en matière active, comprise entre 0,2 % et 5 % en poids, de préférence entre 0,25 % et 3 % en poids, par rapport au poids total de la composition.

Un deuxième composé essentiel des compositions selon l'invention est un polymère du type ester d'acide gras de glycol polyoxyalkyléné ayant des propriétés émulsionnantes eau-dans-huile. L'ester d'acide gras dudit polymère est de préférence polyhydroxylé. En particulier, ce polymère est un polymère séquencé, de préférence de structure ABA, comportant des séquences poly(ester hydroxylé) et des séquences polyéthylèneglycols.

L'ester d'acide gras dudit polymère émulsionnant tel que défini ci-dessus présente en général une chaîne comportant de 12 à 20 atomes de carbone, de préférence de 14 à 18 atomes de carbone. Les esters peuvent notamment être choisis parmi les oléates, les palmitates ou les stéarates.

Les séquences polyéthylèneglycols dudit polymère émulsionnant tel que défini ci-dessus comportent de préférence de 4 à 50 moles d'oxyde d'éthylène, et de préférence encore de 20 à 40 moles d'oxyde d'éthylène.

Un composé convenant particulièrement à la réalisation des compositions de l'invention est le di-polyhydroxystéarate de polyéthylène glycol à 30 OE vendu sous la dénomination commerciale «Arlacel P 135» par la société ICI.

Le polymère du type ester d'acide gras de glycol polyoxyalkyléné est généralement présent dans les compositions selon l'invention à une teneur pouvant aller de 0,2 % à 10 % en poids, par rapport au poids total de la composition, et de préférence de 0,25 % à 5 % en poids, par rapport au poids total de la composition.

Les compositions selon l'invention comprennent également une phase huileuse qui peut comporter un ou plusieurs corps gras, ces corps gras pouvant être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (vaseline), les huiles végétales (huile d'amande douce, de macadamia, de pépin de cassis, de jojoba), les huiles synthétiques comme le perhydrosqualène, les alcools, les acides ou les esters gras (comme le benzoate d'alcools en C₁₂-C₁₅ vendu sous la dénomination commerciale «Finsolv TN» par la société Finetex, le palmitate d'octyle, le lanolate d'isopropyle, les triglycérides dont ceux des acides caprique/caprylique), les esters et éthers gras oxyéthylénés ou oxypropylénés, les huiles fluorées ou encore les polyalkylènes comme le polydécéne.

Comme composés cireux, on peut citer la paraffine, la cire de carnauba, la cire d'abeille, l'huile de ricin hydrogénée.

La phase huileuse peut également comprendre une huile de silicone, volatile ou non telle que les cyclométhicones ou les les diméthicones. On peut par exemple mettre en oeuvre dans les compositions de la présente invention une huile de silicone volatile comme par exemple les cyclométhicones vendues sous les dénominations commerciales «DC 245 Fluid» ou «DC 246 Fluid» par Dow Corning.

Les compositions cosmétiques et/ou dermatologiques visées par la présente invention peuvent bien entendu contenir un ou plusieurs filtres solaires actifs dans l'UVA et/ou l'UVB (absorbeurs), hydrophiles ou lipophiles. Ces filtres peuvent être notamment choisis parmi les dérivés cinnamiques, les dérivés salicyliques, les dérivés du dibenzoylméthane, les dérivés du benzylidène camphre, les dérivés de benzimidazole, les dérivés de triazine, les dérivés de la benzophénone, les dérivés de β,β'-diphénylacrylate, les dérivés de l'acide p-aminobenzoïque, les polymères filtres et silicones filtres décrits dans la demande WO-93/04665. D'autres exemples de filtres organiques sont donnés dans la demande de brevet EP-A 0 487 404.

Les compositions cosmétiques et/ou dermatologiques selon l'invention peuvent encore contenir des pigments ou bien encore des nanopigments (taille moyenne des particules primaires : généralement entre 5 nm et 100 nm, de préférence entre 10 et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs bien connus en soi agissant par blocage physique (réflection et/ou diffusion) du rayonnement UV. Des agents d'enrobage classiques sont par ailleurs l'alumine et/ou le stéarate d'aluminium, les silicones. De tels nanopigments d'oxydes métalliques, enrobés ou non enrobés, sont en particulier décrits dans les demande de brevets EP-A- 0518772 et EP-A- 0518773.

Les nanopigments peuvent être présents dans la composition finale selon l'invention à une teneur pouvant varier de 0,1 à 20 %, de préférence de 0,2 à 10 %, en poids, par rapport au poids total de la composition.

Les compositions selon l'invention peuvent également contenir des épaississants qui peuvent être choisis notamment parmi les acides polyacryliques réticulés, les acides polyacryliques à chaîne grasse, les gommes de guar et celluloses modifiées ou non telles que la gomme de guar hydroxypropylée, la méthylhydroxyéthylcellulose et l'hydroxypropylméthyl cellulose.

Les compositions selon l'invention peuvent également contenir des agents de bronzage et/ou de brunissage artificiels de la peau (agents autobronzants), tels que par exemple de la dihydroxyacétone (DHA).

Les compositions conformes à la présente invention peuvent comprendre en outre des adjuvants cosmétiques et/ou dermatologiques classiques notamment choisis parmi les solvants organiques, les adoucissants, les antioxydants, les agents anti radicaux libres, les opacifiants, les stabilisants, les émollients, les α-hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants, ou tout autre ingrédient habituellement utilisé dans le domaine cosmétique et/ou dermatologique, en particulier pour la fabrication de compositions antisolaires sous forme d'émulsions.

Bien entendu, l'homme de l'art veillera à choisir le ou les éventuels composés complémentaires cités ci-dessus et/ou leurs quantités de manière telle que les propriétés avantageuses attachées intrinsèquement à l'association binaire conforme à l'invention ne soient pas, ou substantiellement pas, altérées par la ou les adjonctions envisagées.

Les compositions selon l'invention peuvent être préparées selon les techniques de préparation d'émulsions de type eau-dans-huile qui sont bien connues de l'homme de l'art.

La composition cosmétique et/ou dermatologique de l'invention peut être utilisée comme composition protectrice de l'épiderme humain ou des cheveux contre les rayons ultraviolets, comme composition antisolaire ou comme produit de maquillage.

Un autre objet de l'invention est l'utilisation de l'association du polyalkyl polyéther siloxane portant des chaînes polyoxyéthylènes et polyoxypropylènes greffées sur la chaîne principale et du polymère du type ester d'acide gras de glycol polyoxyalkyléné à propriétés émulsionnantes eau-dans-huile tels que définis ci-dessus comme système émulsionnant eau-dans-huile, en particulier dans la préparation de compositions cosmétiques et/ou dermatologiques.

Des exemples concrets, mais nullement limitatifs, illustrant l'invention, vont maintenant être donnés.

### EXEMPLE 1 :

La Demanderesse a réalisé des essais comparatifs pour mettre en évidence la sélection réalisée par la présente invention au sein des émulsionnants siliconés eau-dans-huile connus de l'art antérieur, notamment en ce qui concerne la présence nécessaire sur la chaîne principale des polyalkyl siloxanes de groupements oxyéthylénés et oxypropylénés.

On a réalisé les formulations A à E de support commun suivant (les quantités sont exprimées en pourcentage de poids par rapport au poids total de la composition) :
- polyalkyl siloxane 2 %
- di-polyhydroxystéarate de polyéthylène glycol (30 OE) vendu sous la dénomination commerciale « Arlacel P 135 » par ICI 2 %
- benzoate d'alcools en C12/C15 vendu sous la dénomination commerciale « Finsolv TN » par Finetex 6 %
- huile de silicone 16 %
- système filtrant UV 12 %
- oxyde de titane 3 %
- chlorure de sodium 1 %
- glycérol 4 %
- séquestrant 0,3 %
- conservateurs qs
- eau qsp 100 %
à l'exception de la composition B pour laquelle l'huile de silicone (16%) n'a pas été rajoutée, du fait que le produit commercial comprenant le polyalkyl siloxane («DC 3225 C»,cf. tableau (I) ci-dessous) comprend également une huile de silicone (cyclométhicone).

Les compositions respectives des formules A à E en polyalkyl siloxane sont données dans le tableau (I) suivant (les quantités ci-dessous correspondent au pourcentage de produit commercial nécessaire pour obtenir 1,8 % de matière active en pourcentage de poids par rapport au poids total de la composition) :

**Tableau (I) :**

| Formule | Silicone | Taux |
|---|---|---|
| A | « DC Q2-5200 » | 2 % |
| B | « DC 3225 C » | 18 % |
| C | « SILWAX WD-IS » | 1,8 % |
| D | « DC 193 » | 1,8% |
| E | « FANCORSIL LIM-3 » | 1,8 % |

Dans le tableau (I) ci-dessus :
- «DC Q2-5200» est un poly méthyllauryl / méthyl siloxane oxyéthyléné oxypropyléné (35/3) (OE/OP 18/18) (nom CTFA : Laurylméthicone copolyol 91%, Isostéaryl alcohol 9%) fourni par la société Dow Corning (émulsionnant selon l'invention).
- «DC 3225 C» est un poly diméthyl / méthyl siloxane oxyéthyléné oxypropyléné (396/4) (OE/OP 18/18) (nom CTFA : Cyclométhicone 90% Dimethicone copolyol 10%) fourni par la société Dow Corning (émulsionnant selon l'invention).
- «SILWAX WD-I » est un poly diméthylsiloxane oxyéthyléné à groupements isostéarate (nom CTFA : Dimeticone copolyol isostéarate) fourni par la société Siltech (émulsionnant non conforme à l'invention).
- «DC 193» est un poly diméthylsiloxane oxyéthyléné (9/4) (12 OE) (nom CTFA : Diméthicone copolyol) fourni par la société Dow Corning (émulsionnant non conforme à l'invention).
- «FANCORSIL LIM-3» est un poly diméthylsiloxane polyoxyéthyléné à groupements eicosanoate (nom CTFA : Diméthicone copolyol eicosanoate) fourni par la société Fanning Corporation (émulsionnant non conforme à l'invention).

### Mode opératoire :

Les émulsions A à E ont été réalisées à l'aide d'un agitateur à tiges TURBOTEST RAYNERI. La phase grasse contenant entre autres le polyalkyl siloxane et le di-polyhydroxystéarate de polyéthylène glycol a été chauffée à 60 °C. La phase aqueuse a été chauffée à 60 °C séparément. Le pigment a été introduit sous forte agitation dans la phase grasse. On a ensuite incorporé progressivement la phase aqueuse dans la phase grasse en maintenant une agitation forte. On a enfin laissé refroidir l'émulsion obtenue sous agitation modérée jusqu'à température ambiante.

Pour chacune des émulsions ainsi préparées, trois échantillons ont été conservés à l'abri de la lumière respectivement à température ambiante, à 45 °C et à 55°C pendant plusieurs jours.

Les conditions de réalisation et les résultats en stabilité (en jours (j) et heures (h)) sont rassemblés dans le tableau (II) suivant :

**Tableau (II) :**

| Formule | Réalisation | Stabilité à température ambiante | Stabilité à 45 °C | Stabilité à 55 °C |
|---|---|---|---|---|
| A (invention) | facile | 15 j : bonne | 15 j : bonne | 15 j : bonne |
| B (invention) | facile | 15 j : bonne | 15 j : bonne | 15 j : bonne |
| C (comparatif) | difficile | 7 j : instable aspect glaireux | 48 h : instable | 48 h : instable |
| D (comparatif) | impossible | | | |
| E (comparatif) | impossible | | | |

Ces résultats montrent bien que l'association conforme à l'invention, à savoir un polymère du type ester d'acide gras de glycol polyoxyalkyléné ayant des propriétés émulsionnantes eau-dans-huile et un émulsionnant siliconé constitué d'un polyalkyl polyéther siloxane portant des chaînes polyoxyéthylènes et polyoxypropylènes greffées sur la chaîne principale permet d'obtenir une bonne stabilité des émulsions après quinze jours de stockage à 45°C voire même à 55°C contrairement aux émulsions de l'art antérieur contenant le même di-polyhydroxystéarate de polyéthylèneglycol associé à un émulsionnant siliconé classique.

### EXEMPLE 2 :

On donne ci-après un exemple concret d'une composition solaire de type eau-dans-huile conforme à l'invention :
- poly méthyllauryl / méthylsiloxane oxyéthyléné et oxypropyléné (35/3) (18 OE/ 180P) vendu sous la dénomination commerciale «DC Q2-5200» par Dow Corning 2 %
- di-polyhydroxystéarate de polyéthylène glycol (30 OE) vendu sous la dénomination commerciale « Arlacel P 135 » par ICI 2 %
- benzoate d'alcools en C₁₂/C₁₅ vendu sous la dénomination commerciale « Finsolv TN » par Finetex 6 %
- huile de silicone 16 %
- α cyano, β,β'-diphénylacrylate de 2-éthyl hexyle vendu sous la dénomination commerciale « Uvinul N 539 » par BASF 6 %
- oxyde de titane 3 %
- chlorure de sodium 1 %
- glycérol 4 %
- séquestrant 0,3 %
- conservateurs qs
- eau qsp 100 %

Cette composition est particulièrement stable dans les conditions de stockage définies à l'exemple 1 et est également rémanente à l'eau.

## Revendications

1. Emulsion eau-dans-huile caractérisée par le fait qu'elle comprend i) au moins un premier émulsionnant siliconé constitué d'un polyalkyl polyéther siloxane portant des chaînes polyoxyéthylènes et polyoxypropylènes greffées sur la chaîne principale et ii) au moins un deuxième émulsionnant constitué d'un polymère séquencé de structure ABA comportant des séquences poly(ester d'acide gras en C₁₂-C₂₀ hydroxylé) et des séquences polyéthylèneglycols comportant de 4 à 50 moles d'oxyde d'éthylène.

2. Emulsion selon la revendication 1, caractérisée par le fait que l'ester d'acide gras hydroxylé dudit polymère présente une chaîne comportant de 14 à 18 atomes de carbone.

3. Emulsion selon la revendication 1 ou 2, caractérisée par le fait que les séquences polyéthylèneglycols dudit polymère comportent de 20 à 40 moles d'oxyde d'éthylène.

4. Emulsion selon l'une quelconque des revendications précédentes, caractérisée par le fait que ledit polymère séquencé de structure ABA est un di-polyhydroxystéarate de polyéthylène glycol comportant 30 moles d'oxyde d'éthylène.

5. Emulsion selon l'une quelconque des revendications précédentes, caractérisée par le fait que ledit polyalkyl polyéther siloxane répond à la formule générale (I) suivante : 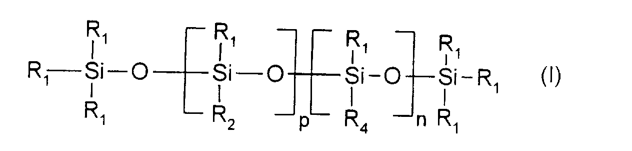 dans laquelle :
- R₁ et R₄, identiques ou différents, représentent un atome d'hydrogène, un radical alkyle linéaire ou ramifié en C₁-C₃₀ ou un radical phényle,
- R₂, identiques ou différents, représentent -(CₓH₂ₓ)-(OC₂H₄)ₐ-(OC₃H₆)_{b}-OR₃,
- R₃, identiques ou différents, sont choisis parmi un atome d'hydrogène, un radical alkyle, linéaire ou ramifié, ayant de 1 à 12 atomes de carbone, un radical acyle, linéaire ou ramifié ayant de 2 à 12 atomes de carbone,
- n varie de 1 à 1000,
- p varie de 1 à 30,
- a varie de 1 à 50,
- b varie de 1 à 50,
- x varie de 1 à 5.

6. Emulsion selon la revendication 5, caractérisée par le fait que les radicaux R₁ et R₄ sont identiques et représentent tous des radicaux méthyle et le radical R₃ représente l'hydrogène.

7. Emulsion selon la revendication 6, caractérisée par le fait que ledit polyalkyl polyéther siloxane est un poly diméthyl / méthyl siloxane oxyéthyléné oxypropyléné de poids moléculaire en nombre supérieur à 30 000 et pour lequel n est 396, p est 4, a est 18 et b est 18.

8. Emulsion selon la revendication 7, caractérisée par le fait que les radicaux R₁ représentent tous des radicaux méthyle et les radicaux R₄ représentent tous des radicaux lauryle.

9. Emulsion selon la revendication 8, caractérisée par le fait que ledit polyalkyl polyéther siloxane est un poly méthyllauryl / méthyl siloxane oxyéthyléné oxypropyléné de poids moléculaire en nombre supérieur à 25 000 et pour lequel n est 35, p est 3, a est 18 et b est 18.

10. Emulsion selon l'une quelconque des revendications précédentes, caractérisée par le fait que l'émulsionnant siliconé est présent dans l'émulsion à une teneur en matière active allant de 0,2 % à 5 % en poids, par rapport au poids total de la composition, de préférence de 0,25 % à 3 % en poids, par rapport au poids total de la composition.

11. Emulsion selon l'une quelconque des revendications précédentes caractérisée par le fait que le polymère séquence de structure ABA est présent dans l'émulsion à une teneur allant de 0,2 % à 10 % en poids, par rapport au poids total de la composition, de préférence de 0,25 % à 5 % en poids, par rapport au poids total de la composition.

12. Utilisation d'une émulsion telle que définie à l'une quelconque des revendications 1 à 11 pour la fabrication de compositions cosmétiques ou dermatologiques destinées à la protection de la peau ou des cheveux contre le rayonnement ultraviolet.

13. Composition cosmétique et/ou dermatologique caractérisée par le fait qu'elle contient au moins une émulsion eau-dans-huile telle que définie à l'une quelconque des revendications 1 à 11.

14. Procédé de traitement cosmétique pour la protection de la peau et/ou des cheveux contre le rayonnement ultraviolet, qui consiste essentiellement à appliquer sur ces derniers une quantité efficace d'une émulsion telle que définie à l'une quelconque des revendications 1 à 11 ou d'une composition telle que définie à la revendication 13.

15. Utilisation de l'association d'un polyalkyl polyéther siloxane portant des chaînes polyoxyéthylènes et polyoxypropylènes greffées sur la chaîne principale tel que défini à l'une quelconque des revendications 1, 5 à 9 et d'un polymère séquencé de structure ABA comportant des séquences poly(ester d'acide gras en C₁₂-C₂₀ hydroxylé) et des séquences polyéthylèneglycols comportant de 4 à 50 moles d'oxyde d'éthylène tel que défini à l'une quelconque des revendications 1 à 4 comme système émulsionnant eau-dans-huile.

## Patentansprüche

1. Wasser-in-Öl-Emulsion, dadurch gekennzeichnet, daß sie i) mindestens einen ersten Siliconemulgator, der aus einem Polyalkylpolyethersiloxan besteht, das auf die Hauptkette gepfropfte polyethoxylierte und polypropoxylierte Ketten aufweist, und ii) mindestens einen zweiten Emulgator enthält, der aus einem Blockpolymer der Struktur ABA besteht, das Poly(hydroxylierter C₁₂₋₂₀-Fettsäureester)-Blöcke und Polyethylenglykol-Blöcke mit 4 bis 50 mol Ethylenoxid aufweist.

2. Emulsion nach Anspruch 1, dadurch gekennzeichnet, daß der hydroxylierte Fettsäureester des Polymers eine Kette mit 14 bis 18 Kohlenstoffatomen aufweist.

3. Emulsion nach Anspruch 1 oder 2, dadurch gekennzeichnet, daß die Polyethylenglykol-Blöcke des Polymers 20 bis 40 mol Ethylenoxid aufweisen.

4. Emulsion nach einem der vorhergehenden Ansprüche, dadurch gekennzeichnet, daß das Blockpolymer der Struktur ABA ein Di-polyhydroxystearat von Polyethylenglykol mit 30 mol Ethylenoxid ist.

5. Emulsion nach einem der vorhergehenden Ansprüche, dadurch gekennzeichnet, daß das Polyalkypolyethersiloxan der folgenden allgemeinen Formel (I) entspricht: 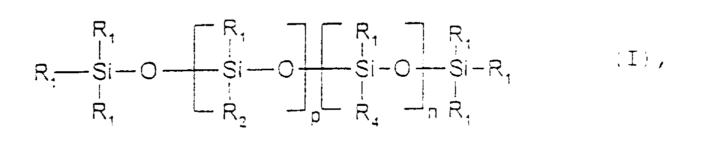 worin:
- die Gruppen R₁ und R₄, die identisch oder voneinander verschieden sind, ein Wasserstoffatom, eine geradkettige oder verzweigte C₁₋₃₀-Alkylgruppe oder eine Phenylgruppe bedeuten,
- die Gruppen-R₂, die identisch oder voneinander verschieden sind, -(CₓH₂ₓ)-(OC₂H₄)ₐ-(OC₃H₆)_{b}-OR₃, bedeuten,
- die Gruppen R₃, die identisch oder voneinander verschieden sind, unter Wasserstorf, einer geradkettigen oder verzweigten Alkylgruppe mit 1 bis 12 Kohlenstoffatomen oder einer geradkettigen oder verzweigten Acylgruppe mit 2 bis 12 Kohlenstoffatomen ausgewählt sind,
- n im Bereich von 1 bis 1000 liegt,
- p im Bereich von 1 bis 30 liegt,
- a im Bereich von 1 bis 50 liegt,
- b im Bereich von 1 bis 50 liegt, und
- x im Bereich von 1 bis 5 liegt.

6. Emulsion nach Anspruch 5, dadurch gekennzeichnet, daß die Gruppen R₁ und R₄ identisch sind und Methylgruppen bedeuten und die Gruppe R₃ Wasserstoff bedeutet.

7. Emulsion nach Anspruch 6, dadurch gekennzeichnet, daß das Polyalkylpolyethersiloxan ein ethoxyliertes und propoxyliertes Polydimethyl/methylsiloxan mit einem Zahlenmittel des Molekulargewichts über 30 000 ist, worin n 396, p 4, a 18 und b 18 bedeutet.

8. Emulsion nach Anspruch 7, dadurch gekennzeichnet, daß die Gruppen R₁ alle Methylgruppen bedeuten und die Gruppen R₄ alle Laurylgruppen bedeuten.

9. Emulsion nach Anspruch 8, dadurch gekennzeichnet, daß das Polyalkylpolyethersiloxan ein ethoxyliertes und propoxyliertes Polymethyllauryl/methylsiloxan mit einem Zahlenmittel des Molekulargewichts über 25 000 ist, worin n 35, p 3, a 18 und b 18 bedeutet.

10. Emulsion nach einem der vorhergehenden Ansprüche, dadurch gekennzeichnet, daß der Siliconemulgator in der Emulsion in einem Wirkstoffanteil im Bereich von 0,2 bis 5 Gew.-%, bezogen auf das Gesamtgewicht der Zusammensetzung, und vorzugsweise von 0,25 bis 3 Gew.-%, bezogen auf das Gesamtgewicht der Zusammensetzung, vorliegt.

11. Emulsion nach einem der vorhergehenden Ansprüche, dadurch gekennzeichnet, daß das Blockpolymer der Struktur ABA in der Emulsion in einem Mengenanteil im Bereich von 0,2 bis 10 Gew.-%, bezogen auf das Gesamtgewicht der Zusammensetzung, und vorzugsweise von 0,25 bis 5 Gew.-%, bezogen auf das Gesamtgewicht der Zusammensetzung, vorliegt.

12. Verwendung einer Emulsion nach einem der Ansprüche 1 bis 11 zur Herstellung von kosmetischen oder dermatologischen Zusammensetzungen, die zum Schutz der Haut oder der Haare gegen UV-Strahlung bestimmt sind.

13. Kosmetische und/oder dermatologische Zusammensetzung, dadurch gekennzeichnet, daß sie mindestens eine Wasserin-Öl-Emulsion nach einem der Ansprüche 1 bis 11 enthält.

14. Verfahren zur kosmetischen Behandlung zum Schutz der Haut und/oder der Haare gegen UV-Strahlung, das im wesentlichen darin besteht, auf diese eine wirksame Menge einer Emulsion nach einem der Ansprüche 1 bis 11 oder einer Zusammensetzung nach Anspruch 13 aufzutragen.

15. Verwendung eines Polyalkylpolyethersiloxans, das auf die Hauptkette gepfropfte polyethoxylierte und polypropoxylierte Ketten aufweist, nach einem der Ansprüche 1 und 5 bis 9 in Kombination mit einem Blockpolymer der Struktur ABA, das Blöcke von Poly(hydroxylierter C₁₂₋₂₀-Fettsäureester) und Polyethylenglykol mit 4 bis 50 mol Ethylenoxid aufweist, nach einem der Ansprüche 1 bis 4 als Wasser-in-Öl-Emulgatorsystem.

## Claims

1. Water-in-oil emulsion, characterized in that it comprises i) at least a first silicone emulsifier composed of a polyalkylpolyethersiloxane carrying polyoxyethylene and polyoxypropylene chains grafted onto the main chain and ii) at least a second emulsifier consisting of a block polymer with an ABA structure containing C₁₂-C₂₀ poly(hydroxylated fatty acid ester) blocks and polyethylene glycol blocks containing from 4 to 50 mol of ethylene oxide.

2. Emulsion according to Claim 1, characterized in that the hydroxylated fatty acid ester of the said polymer exhibits a chain containing from 14 to 18 carbdn atoms.

3. Emulsion according to Claim 1 or 2, characterized in that the polyethylene glycol blocks of the said polymer contain from 20 to 40 mol of ethylene oxide.

4. Emulsion according to any one of the preceding claims, characterized in that the said block polymer with an ABA structure is a polyethylene glycol dipolyhydroxystearate containing 30 mol of ethylene oxide.

5. Emulsion according to any one of the preceding claims, characterized in that the said polyalkylpolyethersiloxane corresponds to the following general formula (I): 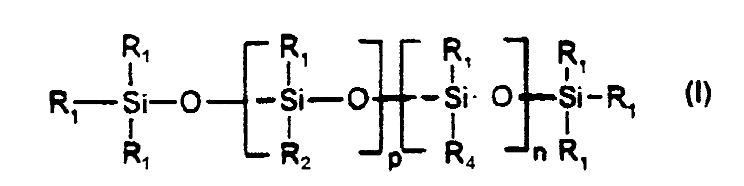 in which:
- R₁ and R₄, which are identical or different, represent a hydrogen atom, a linear or branched C₁-C₃₀ alkyl radical or a phenyl radical,
- R₂, which are identical or different, represent -(CₓH₂ₓ)-(OC₂H₄)ₐ-(OC₃H₆)_{b}-OR₃,
- R₃, which are identical or different, are chosen from a hydrogen atom, a linear or branched alkyl radical having from 1 to 12 carbon atoms or a linear or branched acyl radical having from 2 to 12 carbon atoms,
- n varies from 1 to 1000,
- p varies from 1 to 30,
- a varies from 1 to 50,
- b varies from 1 to 50,
- x varies from 1 to 5.

6. Emulsion according to Claim 5, characterized in that the R₁ and R₄ radicals are identical and all represent methyl radicals and the R₃ radical represents hydrogen.

7. Emulsion according to Claim 6, characterized in that the said polyalkylpolyethersiloxane is a polydimethyl/oxyethylenated oxypropylenated methylsiloxane with a number-average molecular weight of greater than 30,000 and in which n is 396, p is 4, a is 18 and b is 18.

8. Emulsion according to Claim 7, characterized in that the R₁ radicals all represent methyl radicals and the R₄ radicals all represent lauryl radicals.

9. Emulsion according to Claim 8, characterized in that the said polyalkylpolyethersiloxane is a polymethyllauryl/oxyethylenated oxypropylenated methylsiloxane with a number-average molecular weight of greater than 25,000 and in which n is 35, p is 3, a is 18 and b is 18.

10. Emulsion according to any one of the preceding claims, characterized in that the silicone emulsifier is present in the emulsion at an active material content ranging from 0.2% to 5% by weight with respect to the total weight of the composition, preferably from 0.25% to 3% by weight with respect to the total weight of the composition.

11. Emulsion according to any one of the preceding claims, characterized in that the block polymer with an ABA structure is present in the emulsion at a content ranging from 0.2% to 10% by weight with respect to the total weight of the composition, preferably from 0.25% to 5% by weight with respect to the total weight of the composition.

12. Use of an emulsion as defined in any one of Claims 1 to 11 for the manufacture of cosmetic or dermatological compositions intended for the protection of the skin or hair against ultraviolet radiation.

13. Cosmetic and/or dermatological composition, characterized in that it contains at least one water-in-oil emulsion as defined in any one of Claims 1 to 11.

14. Cosmetic treatment method for the protection of the skin and/or hair against ultraviolet radiation, which essentially comprises applying, to the latter, an effective amount of an emulsion as defined in any one of Claims 1 to 11 or of a composition as defined in Claim 13.

15. Use of the combination of a polyalkylpolyethersiloxane carrying polyoxyethylene and polyoxypropylene chains grafted onto the main chain as defined in any one of Claims 1, 5 to 9 and of a block polymer with an ABA structure containing C₁₂-C₂₀ poly(hydroxylated fatty acid ester) blocks and polyethylene glycol blocks containing from 4 to 50 mol of ethylene oxide as defined in any one of Claims 1 to 4 as water-in-oil emulsifying system.
